# EUROPÄISCHE PATENTSCHRIFT

(11) **EP 0 922 689 B1**
(45) Veröffentlichungstag und Bekanntmachung des Hinweises auf die Patenterteilung: **13.11.2002**
(21) Anmeldenummer: 98123446.1
(22) Anmeldetag: 11.12.1998
(51) Int. Cl.: C07C 29/17

(54) **Verfahren zur Hydrierung von Alkinolen unter Verwendung eines Makroporen aufweisenden Katalysators**
Process for the hydrogenation of alkynols using a macropous catalyst
Procédé d'hydrogénation d'alkynols utilisant un catalyseur macroporeux

(30) Priorität: 12.12.1997 DE 19755347
(43) Veröffentlichungstag der Anmeldung: 16.06.1999
(73) Patentinhaber: BASF AKTIENGESELLSCHAFT, 67056 Ludwigshafen (DE)
(72) Erfinder: Böttcher, Arnd, Dr., 67227 Frankenthal (DE); Brunner, Melanie, Dr., 67105 Schifferstadt (DE); Henkelmann, Jochen, Dr., 68165 Mannheim (DE); Rütter, Heinz, Dr., 67126 Hochdorf-Assenheim (DE)
(74) Vertreter: Isenbruck, Günter, Dr.

(56) Entgegenhaltungen:
- EP-A- 0 482 445
- DE-A- 2 145 297

## Beschreibung

Die vorliegende Erfindung betrifft ein Verfahren zur Hydrierung von Alkinolen durch Inkontaktbringen eines oder mehrerer Alkinole mit einem Wasserstoff enthaltenden Gas in Gegenwart eines Makroporen aufweisenden Katalysators.

Aus dem Stand der Technik sind Hydrierungen unter Verwendung der verschiedensten Katalysatoren bekannt.

So beschreibt die DE 195 00 479 die Hydrierung von Acetylenalkoholen mit Pd/Cund Pt/C-Katalysatoren.

In der DE-A 37 17 405 wird ein Katalysator auf der Basis eines Nickelsalzes zur Hydrierung von Alkinolen eingesetzt.

In der US 2 992 278 werden Alkindiole in Gegenwart von Pd, Rh oder Pt in Gegenwart einer geringen Menge Base (z.B. KOH) zu den entsprechenden Alkandiolen hydriert.

Die DE-C 858 094 und die DE-A2 917 018 betreffen die Hydrierung von 1,4-Butindiol in Gegenwart eines Katalysators, der aus Nickel, Kupfer und Mangan auf Silicasträngen besteht. Das dort beschriebene Verfahren wird einstufig durchgeführt. Die Umsetzungstemperaturen betragen 80 bis 160 °C bei einem Druck von 300 bar.

Auch Raney-Katalysatoren (Kupfer, Nickel) werden für derartige Hydrierungen, z.B. in der US 3 449 445, beschrieben. Gemäß dieser Druckschrift wird in einem zweistufigen Verfahren zunächst bei 20 bar über dem suspendierten Raney-Katalysator und in einer zweiten Stufe dann bei 120 - 140 °C über einem Festbettkontakt hydriert.

Die DE-A 2 145 297 offenbart einen Hydrierkatalysator zur Umwandlung von 1,4-Butindiol in 1,4-Butandiol. Dieser Katalysator weist einen großen Oberflächenbereich, auf der Basis von amorphen oder Gamma-Aluminiumoxid mit Anteilen von bis zu 20 Gew.-% Nickel, 10 Gew.-% Kupfer und bis zu 1,5 Gew.-% Mangan, auf. Die Hydrierung wird bei einem Druck von etwa 70 bis etwa 210 kg/cm² und einer Temperatur von etwa 40 °C bis etwa 175 °C durchgeführt.

Die GB-A 20 23 020 betrifft Katalysatoren zur Hydrierung von Butindiol, die als Aktivkomponente sowohl Pd als auch Ru enthalten.

Obwohl, wie sich aus der obigen Zusammenfassung des Standes der Technik ergibt, bereits zahlreiche Katalysatoren zur Hydrierung von Alkinolen eingesetzt wurden, blieben die erreichte Selektivität und Raum-Zeit-Ausbeuten, in denen die entsprechenden Alkanole bzw. Alkandiole erhalten wurden, hinter dem zurück, was wünschenswert wäre, um die immer größeren Erwartungen hinsichtlich der Reinheit der Produkte zu erreichen, bzw. die entsprechenden Verfahren tatsächlich wirtschaftlich durchzuführen.

Demgemäß lag der vorliegenden Erfindung die primäre Aufgabe zugrunde, ein Verfahren zur Hydrierung von Alkinolen zur Verfügung zu stellen, mit Hilfe dessen die entsprechenden Alkanole bzw. Alkandiole mit sehr hoher Selektivität und Raum-Zeit-Ausbeute ohne signifikante Nebenreaktionen erhalten werden können.

Demgemäß betrifft die vorliegende Erfindung ein Verfahren zur Hydrierung eines Alkinols oder eines Gemischs aus zwei oder mehr davon durch Inkontaktbringen des Alkinols oder des Gemischs aus zwei oder mehr davon mit einem Wasserstoff enthaltenden Gas in Gegenwart eines Katalysators, der als Aktivmetall mindestens ein Metall der VIII. Nebengruppe des Periodensystems alleine oder zusammen mit min-4estens einem Metall der I. oder VII. Nebengruppe des Periodensystems, aufgebracht auf einem Träger, umfaßt, dadurch gekennzeichnet, daß der Träger Makroporen aufweist.

In einer bevorzugten Ausführungsform betrifft die vorliegende Erfindung ein Verfahren zur Hydrierung eines Alkinols oder eines Gemischs aus zwei oder mehr davon, wie oben definiert, wobei der Katalysator (Katalysator 1) als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, wobei der Träger einen mittleren Porendurchmesser von mindestens 50 nm und eine Oberfläche BET von höchstens 30 m²/g aufweist und die Menge des Aktivmetalls 0,01 bis 30 Gew.-%, bezogen auf das Gesamtgewicht des Katalysator, beträgt.

Femer betrifft die vorliegende Erfindung in einer bevorzugten Ausführungsform ein Verfahren wie oben definiert, wobei der Katalysator (Katalysator 2) als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Porenvolumina zu 100% addiert.

In einer weiteren bevorzugten Ausführungsform betrifft die vorliegende Erfindung ein Verfahren, wie oben definiert, wobei der Katalysator (Katalysator 3) als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm, und eine Oberfläche BET von höchstens 15 m²/g aufweist.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe "Makroporen" und "Mesoporen" werden im Rahmen der vorliegenden Erfindung so verwendet, wie sie in Pure Appl. Chem., 45, S. 79 (1976) definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen ungefähr 0,01 bis ungefähr 30 Gew.-%, vorzugsweise ungefähr 0,01 bis ungefähr 5 Gew.-% und insbesondere ungefähr 0,1 bis ungefähr 5 Gew.-%, jeweils bezogen auf das Gesamtgewicht des verwendeten Katalysators, wobei die bei den im folgenden beschriebenen, vorzugsweise eingesetzten Katalysatoren 1 bis 3 vorzugsweise verwendeten Gehalte nochmals bei der Diskussion dieser Katalysatoren einzeln angegeben sind.

Der Begriff "Alkinol" umfaßt prinzipiell alle Verbindungen, die sowohl mindestens eine C-C-Dreifachbindung als auch eine oder mehrere Hydroxylgruppen enthalten. Vorzugsweise werden Alkinmonoole, d.h. Verbindungen mit jeweils einer C-C-Dreifachbindung und einer Hydroxylgruppe, oder aber Alkindiole, d.h. Verbindungen mit einer C-C-Dreifachbindung und zwei Hydroxylgruppen, umgesetzt. Die bevorzugt einsetzbaren Verbindungen werden untenstehend im Abschnitt "Die Verfahrensführung" nochmals kurz erläutert.

Im folgenden sollen nunmehr die vorzugsweise verwendeten Katalysatoren 1 bis 3 detailliert beschrieben werden. Dabei erfolgt die Beschreibung beispielshaft unter Bezugnahme auf die Verwendung von Ruthenium als Aktivmetall. Die untenstehenden Angaben sind auch auf die anderen verwendbaren Aktivmetalle, wie hierin definiert, übertragbar.

### KATALYSATOR 1

Die erfindungsgemäß verwendeten Katalysatoren 1 können technisch hergestellt werden durch Auftragen mindestens eines Metalls der VIII. Nebengruppe des Periodensystems und gegebenenfalls mindestens eines Metalls der I. oder VII. Nebengruppe des Periodensystems auf einem geeigneten Träger.

Die Auftragung kann durch Tränken des Trägers in wäßrigen Metallsalzlösungen, wie z.B. wäßrigen Rutheniumsalzlösungen, durch Aufsprühen entsprechender Metallsalzlösungen auf den Träger oder durch andere geeignete Verfahren erreicht werden. Als Metallsalze der I., VII. oder VIII. Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der VIII. Nebengruppe des Periodensystems noch weitere Metalle als Aktivmetall auf dem Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend, vorzugsweise bei Temperaturen zwischen 100 °C und 150 °C, getrocknet und wahlweise bei Temperaturen zwischen 200 °C und 600 °C, vorzugsweise zwischen 350 °C und 450 °C calciniert. Bei getrennter Auftränkung wird der Katalysator nach jedem Tränkschritt getrocknet und wahlweise calciniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen zwischen ungefähr 30 °C und ungefähr 600 °C, vorzugsweise zwischen ungefähr 150 °C und ungefähr 450 °C aktiviert. Vorzugsweise besteht der Gasstrom aus 50 bis 100 Vol.-% H₂ und 0 bis 50 Vol.-% N₂.

Die Metallsalzlösung oder -lösungen werden in einer solchen Menge auf den oder die Träger aufgebracht, daß der Gesamtgehalt an Aktivmetall, jeweils bezogen auf das Gesamtgewicht des Katalysators, ungefähr 0,01 bis ungefähr 30 Gew.-%, vorzugsweise ungefähr 0,01 bis ungefähr 5 Gew.-%, weiter bevorzugt ungefähr 0,01 bis ungefähr 1 Gew.-%, und insbesondere ungefähr 0,05 bis ungefähr 1 Gew.-% beträgt.

Die Metalloberfläche auf dem Katalysator 1 beträgt dabei insgesamt vorzugsweise ungefähr 0,01 bis ungefähr 10 m²/g, weiter bevorzugt ungefähr 0,05 bis ungefähr 5 m²/g und insbesondere ungefähr 0,05 bis ungefähr 3 m²/g des Katalysators. Die Metalloberfläche wird mittels der von J. LeMaitre et al. in "Characterization ofHeterogenous Catalysts", Hrsg. Francis Delanney, Marcel Dekker, New York 1984, S. 310 - 324, beschriebenen Chemisorptionsverfahren bestimmt.

Im erfindungsgemäß verwendeten Katalysator 1 beträgt das Verhältnis der Oberflächen des/der Aktivmetalls/-metalle und des Katalysatorträgers vorzugsweise weniger als ungefähr 0,05, wobei der untere Grenzwert bei ungefähr 0,0005 liegt.

Die zur Herstellung der erfindungsgemäß verwendeten Katalysatoren verwendbaren Trägermaterialien sind solche, die makroporös sind und einen mittleren Porendurchmesser von mindestens ungefähr 50 nm, vorzugsweise mindestens ungefähr 100 nm, insbesondere mindestens ungefähr 500 nm, aufweisen und deren Oberfläche nach BET höchstens ungefähr 30 m²/g, vorzugsweise höchstens ungefähr 15 m²/g, weiter bevorzugt höchstens ungefähr 10 m²/g, insbesondere höchstens ungefähr 5 m²/g und weiter bevorzugt höchstens ungefähr 3 m²/g liegt. Genauer gesagt, beträgt der mittlere Porendurchmesser des Trägers vorzugsweise ungefähr 100 nm bis ungefähr 200 µm, weiter bevorzugt ungefähr 500 nm bis ungefähr 50 µm. Die Oberfläche des Trägers beträgt vorzugsweise ungefähr 0,2 bis ungefähr 15 m²/g, weiter bevorzugt ungefähr 0,5 bis ungefähr 10 m²/g, insbesondere ungefähr 0,5 bis ungefähr 5 m²/g und weiter bevorzugt ungefähr 0,5 bis ungefähr 3 m²/g.

Die Oberfläche des Trägers wird bestimmt nach dem BET-Verfahren durch N₂-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine spezielle Ausführungsform der Erfindung darstellt.

Weiter bevorzugt ist ein Träger mit einer Oberfläche von 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermetall verwendbar sind beispielsweise Aktivkohle, Siliciumcarbid, Aluminiumoxid, Siliciumdioxid, Titandioxid, Zirkoniumdioxid, Magnesiumoxid, Zinkoxid oder Gemische von zwei oder mehr davon, wobei Aluminiumoxid und Zirkoniumdioxid vorzugsweise verwendet werden.

Weitere Details bezüglich Katalysator 1 bzw. zu seiner Herstellung sind der DE-A 196 24 484.6 zu entnehmen, deren diesbezüglicher Inhalt durch Bezugnahme vollständig in die vorliegende Anmeldung einbezogen wird.

### KATALYSATOR 2

Die erfindungsgemäß verwendeten Katalysatoren 2 können technisch hergestellt werden durch Auftragen eines Aktivmetalls der VIII. Nebengruppe des Periodensystems, vorzugsweise Ruthenium oder Palladium und gegebenenfalls mindestens einem Metall der I. oder VII. Nebengruppe des Periodensystems auf einem geeigneten Träger. Die Auftragung kann durch Tränken des Trägers in wäßrigen Metallsalzlösungen, wie Ruthenium- oder Palladiumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw. getränkten Träger werden anschließend getrocknet, wobei Temperaturen zwischen 100 °C und 150 °C bevorzugt sind. Wahlweise können diese Träger bei Temperaturen zwischen 200 °C und 600 °C, vorzugsweise 350 °C bis 450 °C calciniert werden. Anschließend werden die beschichteten Träger durch Behandlung in einem Gasstrom, der freien Wasserstoff enthält, bei Temperaturen zwischen 30 °C und 600 °C, vorzugsweise zwischen 100 °C und 450 °C und insbesondere bei 100 °C bis 300 °C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Auftragen nacheinander, so kann der Träger nach jedem Auftragen bzw. Tränken bei Temperaturen zwischen 100 °C und 150 °C getrocknet werden und wahlweise bei Temperaturen zwischen 200 °C und 600 °C calciniert werden. Dabei kann die Reihenfolge, in der die Metallsalzlösung aufgetragen oder aufgetränkt werden, beliebig gewählt werden.

Die Metallsalzlösung wird in einer solchen Menge auf den/die Träger aufgebracht, daß der Gehalt an Aktivmetall 0,01 bis 30 Gew.-%, vorzugsweise 0,01 bis 10 Gew.-% und insbesondere 0,01 bis 5 Gew.-% und insbesondere 0,3 bis 1 Gew.-%, bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. LeMaitre et al., *"Characterization of Heterogeneous Catalysts"*, Hrsg. Francis Delanney, Marcel Dekker, New York (1984), S. 310- 324, beschrieben ist.

Im erfindungsgemäß verwendeten Katalysator 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die zur Herstellung der erfindungsgemäß verwendeten Katalysatoren 2 verwendbaren Trägermaterialien besitzen Makroporen und Mesoporen.

Dabei weisen die erfindungsgemäß verwendbaren Träger eine Porenverteilung auf, der gemäß ungefähr 5 bis ungefähr 50%, vorzugsweise ungefähr 10 bis ungefähr 45%, weiter bevorzugt ungefähr 10 bis ungefähr 30% und insbesondere ungefähr 15 bis ungefähr 25% des Porenvolumens von Makroporen mit Porendurchmesser im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 85% des Porenvolumens von Mesoporen mit einem Porendurchmesser von ungefähr 2 bis ungefähr 50 nm gebildet werden, wobei sich jeweils die Summe der Porenvolumina zu 100% addiert.

Das Gesamtporenvolumen der erfindungsgemäß verwendeten Träger beträgt ungefähr 0,05 bis 1,5 cm³/g, vorzugsweise 0,1 bis 1,2 cm³/g und insbesondere 0,3 bis 1,0 cm³/g. Der mittlere Porendurchmesser der erfindungsgemäß verwendeten Träger beträgt ungefähr 5 bis 20 nm, vorzugsweise ungefähr 8 bis 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 200 bis ungefähr 250 m²/g des Trägers.

Die Oberfläche des Trägers wird nach dem BET-Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich Katalysator 2 bzw. zu seiner Herstellung sind der DE-A 196 24 485.4 zu entnehmen, deren diesbezüglicher Inhalt durch Bezugnahme vollständig in die vorliegende Anmeldung einbezogen wird.

### KATALYSATOR 3

Die erfindungsgemäß verwendeten Katalysatoren 3 können technisch hergestellt werden durch Auftragen eines Aktivmetalls der VIII. Nebengruppe des Periodensystems und gegebenenfalls mindestens einem Metall der I. oder VII. Nebengruppe des Periodensystems auf einen geeigneten Träger. Die Auftragung kann durch Tränken des Trägers in wäßrigen Metallsalzlösungen, wie z.B. Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I., VII. oder VIII. Nebengruppe eignen sich die Nitrate, Nitrosylnitrate, Halogenide, Carbonate, Carboxylate, Acetylacetonate, Chlorkomplexe, Nitritokomplexe oder Aminkomplexe der entsprechenden Metalle, bevorzugt sind dabei die Nitrate und Nitrosylnitrate.

Bei Katalysatoren, die mehrere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen zwischen 100 °C und 150 °C, und wahlweise bei Temperaturen zwischen 200 °C und 600 °C caiciniert.

Darauffolgend werden die beschichteten Träger aktiviert durch Behandlung der beschichteten Träger in einem Gasstrom, der freien Wasserstoff enthält, bei Temperaturen zwischen 30 und 600 °C, vorzugsweise zwischen 150 und 450 °C. Der Gasstrom besteht vorzugsweise aus 50 bis 100 Vol-% H₂ und 0 bis 50 Vol-% N₂.

Werden auf die Träger neben dem Aktivmetall der VIII. Nebengruppe des Periodensystems Metalle der I. oder VII. Nebengruppe aufgetragen und erfolgt das Auftragen nacheinander, so kann der Träger nach jedem Auftragen bzw. Tränken bei Temperaturen zwischen 100 und 150 °C getrocknet werden und wahlweise bei Temperaturen zwischen 200 und 600 °C calciniert werden. Dabei kann die Reihenfolge, in der die Metallsalzlösungen aufgetragen oder aufgetränkt werden, beliebig gewählt werden.

Die Metallsalzlösung wird in einer solchen Menge auf den oder die Träger aufgebracht, daß 0,01 bis 30 Gew.-%, bezogen auf das Gesamtgewicht des Katalysators, an Aktivmetall auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator 3 beträgt insgesamt vorzugsweise 0,01 bis 10 m²/g, besonders bevorzugt 0,05 bis 5 m²/g, insbesondere 0,05 bis 3 m² pro g des Katalysators.

Die zur Herstellung der erfindungsgemäß verwendeten Katalysatoren 3 verwendbaren Trägermaterialien sind vorzugsweise solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g. Bevorzugt liegt der mittlere Porendurchmesser des Trägers in einem Bereich von 0,1 bis 200 µm, insbesondere 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m² pro g des Trägers.

Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesserverteilung mit Maxima bei etwa 0,6 µm und etwa 20 µm bei der bimodalen Verteilung eine spezielle Ausführungsform der Erfindung darstellt.

Besonders bevorzugt ist ein Träger mit einer Oberfläche von etwa 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Aktivkohle, Siliciumcarbid, Aluminiumoxid, Siliciumdioxid, Titandioxid, Zirkoniumdioxid, Magnesiumoxid, Zinkoxid oder deren Gemische. Bevorzugt sind Aluminiumoxid und Zirkoniumdioxid.

Weitere Details bezüglich Katalysator 3 bzw. zu seiner Herstellung sind der DE-A 196 04 791.9 zu entnehmen, deren diesbezüglicher Inhalt durch Bezugnahme vollständig in die vorliegende Anmeldung einbezogen wird.

### DIE VERFAHRENSFÜHRUNG

Im Rahmen des erfindungsgemäßen Verfahrens wird die Hydrierung im allgemeinen bei einer Temperatur von ungefähr 50 bis 250 °C, vorzugsweise ungefähr 70 bis 220 °C durchgeführt. Die dabei verwendeten Drücke liegen in der Regel bei oberhalb von 10 bar, vorzugsweise ungefähr 20 bis ungefähr 300 bar.

Das erfindungsgemäße Verfahren kann entweder kontinuierlich oder diskontinuierlich durchgeführt werden, wobei die kontinuierliche Verfahrensdurchführung bevorzugt ist.

Bei der kontinuierlichen Verfahrensführung beträgt die Menge des zur Hydrierung vorgesehenen Alkinols bzw. des Gemischs aus zwei oder mehr davon vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die erfindungsgemäßeHydrierung kann in Ab- oder Anwesenheit eines Lösungsoder Verdünnungsmittels durchgeführt werden, d.h. es ist nicht erforderlich, die Hydrierung in Lösung durchzuführen.

Vorzugsweise wird jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit dem zu hydrierenden Alkinol eine homogene Lösung zu bilden. Beispielsweise können die Lösungsoder Verdünnungsmittel auch Wasser enthalten.

### Beispiele geeigneter Lösungs- oder Verdünnungsmittel schließen die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome aufweist.

Beispiele bevorzugt verwendbarer Alkohole sind i-Propanol, n-Butanol, i-Butanol und n-Hexanol.

Gemische dieser oder anderer Lösungs- oder Verdünnungsmittel können ebenfalls verwendet werden.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 gew.-%igen Lösung des zur Hydrierung vorgesehenen Alkinols führen.

Besonders bevorzugt wird im Rahmen des erfindungsgemäßen Verfahrens das bei der Hydrierung gebildete Produkt, also das Alkanol, als Lösungsmittel eingesetzt, gegebenenfalls neben anderen Lösungs- oder Verdünnungsmitteln. In jedem Fall kann ein Teil des im Verfahren gebildeten Produkts dem noch zu hydrierenden Alkinol beigemischt werden. Bezogen auf das Gewicht der zur Hydrierung vorgesehenen Verbindung wird vorzugsweise die 1- bis 30-fache, besonders bevorzugt die 5- bis 20-fache, insbesondere die 5- bis 10-fache Menge des Umsetzungsproduktes als Lösungs- oder Verdünnungsmittel zugemischt.

Wie oben bereits ausgeführt, umfaßt der erfindungsgemäß verwendete Begriff "Alkinol" sowohl Alkinmonoole als auch Alkindi- bzw. Alkinpolyole, wobei vorzugsweise Alkindiole umgesetzt werden.

### Beispielhaft zu nennen sind insbesondere:

Propargylalkohol, 2,3-Butin-1-ol, 1,4-Butindiol, 1,6-Hexindiol, 2,5-Dimethylhexindiol, 1,8-Octindiol, Dehydrolinalool oder ein Gemisch aus zwei oder mehr davon.

Im folgenden soll nunmehr das erfindungsgemäße Verfahren anhand einiger Ausführungsbeispiele näher erläutert werden.

### BEISPIELE

### Herstellungsbeispiel

Ein meso-/makroporöser Aluminiumoxidträger in Form von 4 mm-Extrudaten, der eine BET-Oberfläche von 238 m²/g und ein Porenvolumen von 0,45 ml/g besaß, wurde mit einer wäßrigen Ruthenium-(III)-nitrat-Lösung, die eine Konzentration von 0,8 Gew.-% aufwies, getränkt. 0,15 ml/g (ungefähr 33% des Gesamtvolumens) der Poren des Trägers besaßen einen Durchmesser im Bereich von 50 nm bis 10.000 nm und 0,30 ml/g (ungefähr 67% des Gesamt Porenvolumens) der Poren des Trägers wiesen einen Porendurchmesser im Bereich von 2 bis 50 nm auf. Das während des Tränkens vom Träger aufgenommene Lösungsvolumen entsprach dabei in etwa dem Porenvolumen des verwendeten Trägers.

Anschließend wurde der mit der Ruthenium-(III)-nitrat-Lösung getränkte Träger bei 120 °C getrocknet und bei 200 °C im Wasserstrom aktiviert (reduziert). Der so hergestellte Katalysator enthielt 0,05 Gew.-% Ruthenium, bezogen auf das Gewicht des Katalysators.

### Beispiel 1

In einem 300 ml-Druckreaktor wurden 10 g des geträgerten Ru-Katalysators in einem Katalysator-Korbeinsatz vorgelegt und mit 160 g (0,33 mol) einer 30%-igen Lösung von 2,5-Dimethylhexindiol in Isopropanol versetzt. Die Hydrierung wurde mit reinem Wasserstoff bei einem konstanten Druck von 30 bar und einer Temperatur von 140 °C durchgeführt. Es wurde solange hydriert, bis kein Wasserstoff mehr aufgenommen wurde (2,5 h). Anschließend wurde der Reaktor entspannt. Der Umsatz des Alkins betrug 100 %. Die Ausbeute an 2,5-Dimethylhexandiol lag bei 97 %, bezogen auf die eingesetzte Menge an 2,5-Dimethylhexindiol.

### Beispiel 2

Ein senkrecht stehendes Reaktionsrohr aus Edelstahl mit einem inneren Durchmesser von 30 mm und einer Länge von 1,7 m wurde mit 800 ml des Ru-Katalysators gemäß Herstellungsbeispiel gefüllt. Im Falle der Sumpffahrweise wurden 0,8 kg/h einer 30%-igen Lösung von 2,5-Dimethylhexindiol in Isobutanol mit reinem Wasserstoff bei einer mittleren Temperatur von 130 °C und einem Druck von 30 bar von unten nach oben durch das senkrecht stehende Reaktionsrohr gepumpt. Ein Teil des Reaktionsproduktes wurde nach Verlassen des senkrecht stehenden Reaktionsrohrs zusammen mit neuer 2,5-Dimethylhexindiol-Lösung erneut in den Reaktor gepumpt. Das restliche Reaktionsprodukt wurde in einem Auffangbehälter entspannt. Abgaskontrolliert wurde mit einem 10%-igen Überschuß des theoretisch benötigten Wasserstoffs hydriert.

Die gaschromatographische Analyse des Reaktionsaustrages zeigt, daß das Alkin zu 100% umgesetzt wurde. 2,5-Dimethylhexandiol konnte mit einer Selektivität von 97%, bezogen auf die eingesetzte Menge an 2,5-Dimethylhexindiol, erhalten werden. Die Katalysatorstandzeit unter den gewählten Bedingungen betrug mindestens 2200h, ohne daß eine Aktivitäts- und Selektivitätsabnahme beobachtet werden konnte.

### Beispiel 3

In einem 300 ml-Druckreaktor wurden 10 g des Ru-Katalysators gemäß Herstellungsbeispiel in einem Katalysator-Korbeinsatz vorgelegt und mit 150 g (0,87 mol) einer 50%-igen wäßrigen Butindiol-Lösung versetzt. Die Hydrierung wurde mit reinem Wasserstoff bei einem konstanten Druck von 40 bar und einer Temperatur von 80 °C durchgeführt. Es wurde solange hydriert, bis kein Wasserstoff mehr aufgenommen wurde (3 h). Anschließend wurde der Reaktor entspannt. Der Umsatz des Alkins betrug 100%. Die Ausbeute an 1,4-Butandiol lag bei 92% bezogen auf die eingesetzte Menge an Butindiol.

### Beispiel 4

In einem 300 ml-Druckreaktor wurden 10 g des Ru-Katalysators gemäß Herstellungsbeispiel in einem Katalysator-Korbeinsatz vorgelegt und mit 150 g (0,99 mol) Dehydrolinalool versetzt. Die Hydrierung wurde mit reinem Wasserstoff bei einem konstanten Druck von 40 bar und einer Temperatur von 80 °C durchgeführt. Nach 4 Stunden Reaktionszeit wurde der Reaktor entspannt. Der Umsatz von Dehydrolinalool betrug 95%. Linalool wurde mit einer Selektivität von 90% erhalten.

Wie sich aus den Beispielen ergibt, ist es mit dem erfindungsgemäßen Verfahren möglich, mit der hohen Selektivität und hoher Raum-Zeit-Ausbeute Alkinole zu den entsprechenden Alkanolen bzw. Alkandiolen umzusetzen. Ferner werden keine signifikanten Nebenreaktionen beobachtet. Die verwendeten Katalysatoren weisen darüber hinaus eine überaus lange Standzeit auf. Ein weiterer Vorteil des erfindungsgemäßen Verfahrens ist darin zu sehen, daß die Hydrierung auch bei verhältnismäßigen niedrigen Drücken von z.B. 20 bis 50 bar durchgeführt werden kann, so daß insgesamt vergleichsweise geringe Investitionskosten für die zu verwendenden Druckapparaturen anfallen.

## Patentansprüche

1. Verfahren zur Hydrierung eines Alkinols oder eines Gemischs aus zwei oder mehr davon durch Inkontaktbringen des Alkinols oder des Gemischs aus zwei oder mehr davon mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, **dadurch gekennzeichnet, daß** der Träger Poren aufweist, deren Porengröße oberhalb 50 nm liegt.

2. Verfahren nach Anspruch 1, **dadurch gekennzeichnet, daß** der Katalysator als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, wobei der Träger einen mittleren Porendurchmesser von mindestens 50 nm und eine Oberfläche BET von höchstens 30 m²/g aufweist und die Menge des Aktivmetalls 0,01 bis 30 Gew.- %, bezogen auf das Gesamtgewicht des Katalysator, beträgt.

3. Verfahren nach Anspruch 1, **dadurch gekennzeichnet, daß** der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Porenvolumina zu 100% addiert.

4. Verfahren nach Anspruch 1, **dadurch gekennzeichnet, daß** der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einen Träger, umfaßt, wobei der Träger einen mittleren Porendurchmesser von mindestens 0,1 µm, - und eine Oberfläche BET von höchstens 15 m²/g aufweist.

5. Verfahren nach einem der Ansprüche 1 bis 4, **dadurch gekennzeichnet, daß** das Alkinol ausgewählt wird aus der Gruppe bestehend aus Propargylalkohol, 2,3-Butin-1-ol, 1,4-Butindiol, 1,6-Hexindiol, 2,5-Dimethylhexindiol, 1,8-Octindiol, Dehydrolinalool und einem Gemisch aus zwei oder mehr davon.

6. Verfahren nach einem der vorstehenden Ansprüche, **dadurch gekennzeichnet, daß** der Träger Aktivkohle, Siliciumcarbid, Aluminiumoxid, Siliciumdioxid, Titandioxid, Zirkoniumdioxid, Magnesiumoxid, Zinkoxid oder ein Gemisch aus zwei oder mehr davon enthält.

7. Verfahren nach einem der vorstehenden Ansprüche, **dadurch gekennzeichnet, daß** die Hydrierung in Gegenwart eines Lösungs- oder Verdünnungsmittels durchgeführt wird.

8. Verfahren nach einem der vorstehenden Ansprüche, **dadurch gekennzeichnet, daß** die Hydrierung kontinuierlich durchgeführt wird.

## Claims

1. A process for hydrogenating an alkynol or a mixture of two or more thereof by bringing the alkynol or the mixture of two or more thereof into contact with a hydrogen-containing gas in the presence of a catalyst comprising as active metal at least one metal of transition group VIII of the Periodic Table, either alone or together with at least one metal of transition group I or VII of the Periodic Table, applied to a support, wherein the support contains pores whose pore size is above 50 nm.

2. A process as claimed in claim 1, wherein the catalyst comprises as active metal at least one metal of transition group VIII of the Periodic Table, either alone or together with at least one metal of transition group I or VII of the Periodic Table, applied to a support, wherein the support has a mean pore diameter of at least 50 nm and a BET surface area of not more than 30 m²/g and the amount of active metal is from 0.01 to 30% by weight, based on the total weight of the catalyst.

3. A process as claimed in claim 1, wherein the catalyst comprises as active metal at least one metal of transition group VIII of the Periodic Table, either alone or together with at least one metal of transition group I or VII of the Periodic Table, in an amount of from 0.01 to 30% by weight, based on the total weight of the catalyst, applied to a support, wherein from 10 to 50% of the pore volume of the support is made up by macropores having a pore diameter in the range from 50 nm to 10,000 nm and from 50 to 90% of the pore volume of the support is made up by mesopores having a pore diameter in the range from 2 to 50 nm, where the sum of the pore volumes adds up to 100%.

4. A process as claimed in claim 1, wherein the catalyst comprises as active metal at least one metal of transition group VIII of the Periodic Table, either alone or together with at least one metal of transition group I or VII of the Periodic Table, in an amount of from 0.01 to 30% by weight, based on the total weight of the catalyst, applied to a support, wherein the support has a mean pore diameter of at least 0.1 ·m and a BET surface area of not more than 15 m²/g.

5. A process as claimed in any of claims 1 to 4, wherein the alkynol is selected from the group consisting of propargyl alcohol, 2,3-butyn-1-ol, 1,4-butynediol, 1,6-hexynediol, 2,5-dimethylhexynediol, 1,8-octynediol, dehydrolinalool and mixtures of two or more thereof.

6. A process as claimed in any of the preceding claims, wherein the support comprises activated carbon, silicon carbide, aluminum oxide, silicon dioxide, titanium dioxide, zirconium dioxide, magnesium oxide, zinc oxide or a mixture of two or more thereof.

7. A process as claimed in any of the preceding claims, wherein the hydrogenation is carried out in the presence of a solvent or diluent.

8. A process as claimed in any of the preceding claims, wherein the hydrogenation is carried out continuously.

## Revendications

1. Procédé d'hydrogénation d'un alcynol ou d'un mélange de deux ou de plusieurs de ceux-ci, au moyen de la mise en contact de l'alcynol ou du mélange de deux ou de plusieurs de ceux-ci avec un gaz contenant de l'hydrogène, en présence d'un catalyseur, qui comprend, en tant que métal actif, au moins un métal du sous-groupe VIII de la Classification Périodique des Eléments, seul ou en combinaison avec au moins un métal du sous-groupe I ou VII de la Classification Périodique des Eléments, placé sur un support, **caractérisé en ce que** le support présente des pores, dont la taille de pores est supérieure à 50 nm.

2. Procédé selon la revendication 1, **caractérisé en ce que** le catalyseur comprend, en tant que métal actif, au moins un métal du sous-groupe VIII de la Classification Périodique des Eléments, seul ou en combinaison avec au moins un métal du sous-groupe I ou VII de la Classification Périodique des Eléments, placé sur un support, dans lequel le support présente un diamètre de pore moyen d'au moins 50 nm et une surface BET inférieure à 30 m²/g et dans lequel la quantité du métal actif est comprise entre 0,01% et 30% en poids, par rapport au poids total du catalyseur.

3. Procédé selon la revendication 1, **caractérisé en ce que** le catalyseur comprend, en tant que métal actif, au moins un métal du sous-groupe VIII de la Classification Périodique des Eléments, seul ou en combinaison avec au moins un métal du sous-groupe I ou VII de la Classification Périodique des Eléments, en une quantité allant de 0,01% à 30% en poids, par rapport au poids total du catalyseur, placé sur un support, dans lequel de 10% à 50% du volume de pores du support est formé par des macropores, dont le diamètre de pore est compris dans la gamme allant de 50 nm à 10 000 nm, et de 50% à 90% du volume de pores du support est formé par des mésopores, dont le diamètre de pore est compris dans la gamme allant de 2 nm à 50 nm, dans lequel la somme des volumes de pores est de 100%.

4. Procédé selon la revendication 1, **caractérisé en ce que** le catalyseur comprend, en tant que métal actif, au moins un métal du sous-groupe VIII de la Classification Périodique des Eléments, seul ou en combinaison avec un métal du sous-groupe I ou VII de la Classification Périodique des Eléments, en une quantité allant de 0,01% à 30% en poids, par rapport au poids total du catalyseur, placé sur un support, dans lequel le support présente un diamètre de pore moyen d'au moins 0,1 µm et une surface BET inférieure à 15 m²/g.

5. Procédé selon l'une quelconque des revendications 1 à 4, **caractérisé en ce que** l'on choisit l'alcynol dans le groupe composé de l'alcool propargylique, du 2,3-butyn-1-ol, du 1,4-butyndiol, du 1,6-hexyndiol, du 2,5-diméthylhexyndiol, du 1,8-octyndiol, du déhydrolinalool et d'un mélange de deux ou de plusieurs d'entre eux.

6. Procédé selon l'une quelconque des revendications précédentes, **caractérisé en ce que** le support contient du charbon actif, du carbure de silicium, de l'oxyde d'aluminium, du dioxyde de silicium, du dioxyde de titane, du dioxyde de zirconium, de l'oxyde de magnésium, de l'oxyde de zinc ou un mélange d'un ou de plusieurs d'entre eux.

7. Procédé selon l'une quelconque des revendications précédentes, **caractérisé en ce que** l'on réalise l'hydrogénation en présence d'un solvant ou d'un diluant.

8. Procédé selon l'une quelconque des revendications précédentes, **caractérisé en ce que** l'on réalise l'hydrogénation en continu.
